(19) Europäisches Patentamt
European Patent Office
Office européen des brevets

(11) Publication number: **0 266 161 B1**

(12) **EUROPEAN PATENT SPECIFICATION**

(45) Date of publication of patent specification: **22.01.92**

(51) Int. Cl.$^5$: **C07C 409/16**, C08K 5/14

(21) Application number: **87309456.9**

(22) Date of filing: **26.10.87**

The file contains technical information submitted after the application was filed and not included in this specification

(54) **Liquid organic peroxide compositions.**

(30) Priority: **29.10.86 IT 2216786**

(43) Date of publication of application:
**04.05.88 Bulletin 88/18**

(45) Publication of the grant of the patent:
**22.01.92 Bulletin 92/04**

(84) Designated Contracting States:
**BE CH DE ES FR GB LI NL SE**

(56) References cited:
**DE-A- 2 912 061**
**DE-A- 2 942 362**
**US-A- 3 764 628**
**US-A- 4 450 302**

**CHEMICAL ABSTRACTS, vol. 95, no. 22, November 30, 1981, Columbus, Ohio, USA; RAPCHINSKAYA et al.: "Study of the vulcanization of saturated polymers by peroxides with sequential distribution of peroxy groups in the molecule", page 57,column 2, abstract no. 188 410x**

(73) Proprietor: **AUSIMONT S.p.A.**
**31, Foro Buonaparte**
**I-20121 Milano(IT)**

(72) Inventor: **Pagliari, Alberto**
**29, piazza Unita d'Italia**
**D-21047 Saronno (Varese)(IT)**
Inventor: **Scotti, Carlo**
**38, via Piemonte**
**D-27058 Voghera (Pavia)(IT)**
Inventor: **Del Bianco, Roberto**
**16, via Barzini**
**I-20125 Milano(IT)**
Inventor: **Angeloni, Giorgio**
**119, via Ripa di P.ta Ticinese 20100**
**I-Milano(IT)**
Inventor: **Merenda, Michele**
**5, via Alessandria**
**I-15065 Frugarolo, Alessandria(IT)**

(74) Representative: **Whalley, Kevin et al**
**MARKS & CLERK 57/60 Lincoln's Inn Fields**
**London WC2A 3LS(GB)**

EP 0 266 161 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention is concerned with liquid organic peroxide compositions suitable for use in the cross-linking of polymers such as polyethylene or ethylene/propylene copolymers.

Italian Patent 1,114,215 describes liquid peroxide compositions, particularly useful for the cross-linking of polymers (for instance polyethylene or ethylene-propylene elastomers), containing dicumyl peroxide (hereinafter simply referred to as "DCP"). The use of DCP alone is limited since it is a solid at room temperature and hence it is necessary that for more uniform metering (in a continuous operation) the DCP be kept molten, with consequent possible loss of peroxide activity and the introduction of impurities. The above-mentioned Italian Patent therefore suggests the use of balanced peroxy-mixtures, containing from 5 to 75% of DCP together with peroxides which give rise to the formation of liquid mixtures and are of the formula:

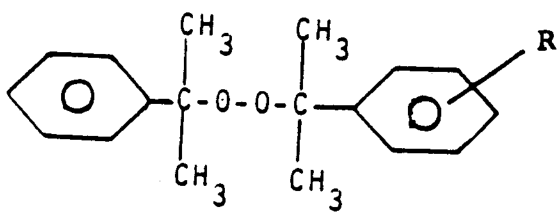

(I)

in which R, which is in the meta or para position, is a $C_1$-$C_3$ alkyl group. In particular, peroxides where R is methyl are recommended, whereas according to U.S.-A-4,202,790, it is more advantageous to use a peroxide wherein R is an isopropyl group, namely isopropylcumyl-cumyl peroxide, provided that the amount of DCP in the peroxide mixture is equal to or higher than 25% and preferably 40% by weight. The U.S. patent proposes separate prepartion of the peroxides (at temperatures above room temperature) and subsequent mixing with DCP in the desired ratio.

Attention is also drawn to the disclosures of US-A-4 450 302 and of US-A-3 764 628.

We have now found that certain peroxide mixtures, as hereinafter defined, give rise to better results, in comparison with previously proposed mixtures, and can be prepared and treated in a much simpler and more practical way.

In accordance with one embodiment the invention provides a liquid peroxide composition containing:

(a) from 1 to 50 parts by weight of a diperoxide of the formula:

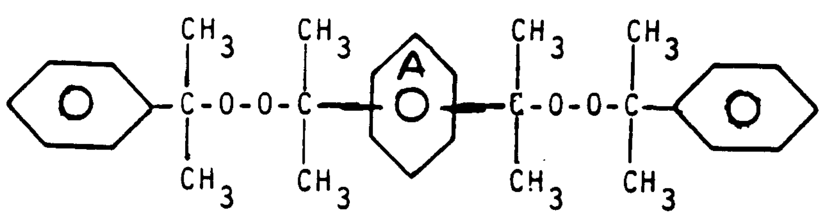

(II)

in which the two substituent groups on the central aromatic ring A are in the meta- and/or para-position and the meta/para isometric ratio is preferably from 1.2 :1 to 2.5 :1, more preferably from 1.5 : 1 to 2.1 : 1 (the peroxide commercially known as "PEROXIMON 169");

(b) from 5 to 75 parts by weight of dicumyl-peroxide (DCP); and (c) from 1 to 85 parts by weight of a peroxide of the formula:

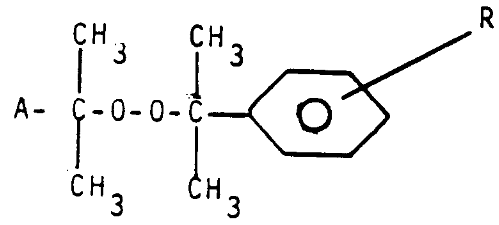

(III)

in which $R^1$ is an isopropyl group, and A is a methyl or phenyl group.

The group R can be in the meta and/or para position, the meta:para isomeric ratio preferably being from 1.2 : 1 to 2.5 : 1 and more preferably from 1.5 : 1 to 2.1 : 1.

The peroxides of formula (II) are described in U.S.-A- 3,787,504 and US-A-3,118,866.

The compositions of the invention differ from DCP in that they are liquid at room temperature and therefore can be incorporated into a polymer without any previous melting; furthermore, they can be easily proportioned and any mixing operation can be performed in a safe manner. As a result, uniformly finished articles can be easily obtained.

Unlike the previously known products of formula (I), the diperoxide of formula (II) has a cross-linking power (on polyethylene) greater than that of DCP and therefore it is not necessary to use increased amounts of peroxide composition, in contrast to mixtures containing DCP and peroxides of formula (I), where between 20 and 35% by weight more of the mixture is generally used.

The compositions of the invention are liquid and therefore it is possible to more easily avoid the presence of impurities; in fact it is easier to detect and remove extraneous substances if present.

The cross-linking efficiency of the compositions of the invention is comparable with that which can be obtained using pure DCP and as the mixing of liquid compositions with a polymer (e.g. in an extruder) can be easily effected, the working of the polymer is simplified. Polymers which can be cross-linked with the compositions of the invention are generally olefin and vinyl thermoplastic polymers, and elastomeric polymers. More particularly there may be mentioned middle, low and high density polyethylene, poly-butene-1, ethylene/vinyl acetate copolymers, acrylic ester/ethylene copolymers, ethylene/propylene copolymers, ethylene/butene-1 copolymers, ethylene/4-methylpentene-1 copolymers and propylene/butene-1 copolymers. In addition, there may be mentioned elastomeric polymers or copolymers such as for instance ethylene/propylene copolymers of the EP or EPDM type, butyl rubber, chlorinated polyethylene and propylene/butene-1 copolymers.

Further, mixtures of two or more olefinic thermoplastic polymers, mixtures of two or more elastomers and mixtures of one or more olefinic thermoplastic polymers with one or more elastomeric polymers can be successfully cross-linked. The compositions of the invention can be used not only for the cross-linking of compact articles, obtained by extrusion or moulding, but also for producing expanded cross-linked articles derived from the same materials, in particular from polystyrene containing self-extinguishing agents (antiflammatory agents). The compositions of the invention can also be used to promote the decomposition of polymers which are decomposed by peroxides (for instance polypropylene or poly-4-methyl-pentene-1) and as free radical polymerization initiators.

The compositions of the invention may be prepared by various methods. In a preferred process, benzene is alkylated with an excess of propylene, in the presence of $AlCl_3$ and according to generally known techniques such as the Friedel-Crafts reaction, to give a di-isopropylbenzenic mixture (prevalently meta and para). The resultant mixture is then oxidized, using generally known techniques, until a predetermined amount of di-hydroperoxide, corresponding to the desired amount of diperoxide of formula (II), is obtained in addition to mono-hydroperoxide. The resulting oxidized mixture, containing unreacted hydrocarbons, as well as monohydroperoxides and dihydroperoxides, is then reduced, for instance by the use of sulfides, sulfites or sulfohydroxides (according to generally used techniques), so that the hydroperoxy groups are converted to alcoholic groups (hydroxy groups). The reduced mixture obtained is uncompletely distilled to give a rectified mixture containing a mono-alcohol of the formula:

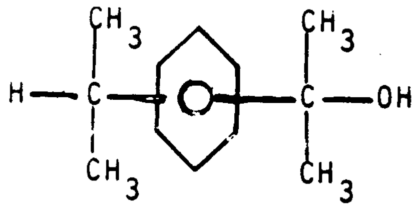

(VI)

and up to 30% by weight of a di-alcohol of the formula:

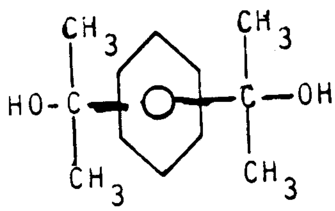

(IV)

together with lower amounts (up to 15%) of unconverted hydrocarbons. Depending on the composition of the desired peroxide mixture, there are then added, in some cases, amounts of particular alcohols (the same as or different from the preceding ones), in particular cumyl alcohol.

The mixture containing the alcohols, however prepared, is then reacted with a cumene mixture (deriving, for instance, from the intermediate step of a conventional plant for the synthesis of phenol), containing higher amounts of cumene hydroperoxide and up to 10% by weight (based on the weight of hydroperoxide) of cumyl alcohol:

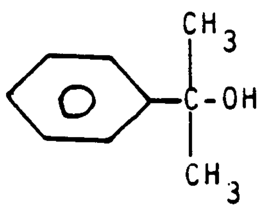

(V)

(a common component of cumenic mixtures) in the presence of one of the commonly used acid catalysts (such as described in U.S. Patents Nos. 4,266, 081; 4,239,644; 4,202,790; and 2,668,180) and under suitable conditions, for example as described in these U.S. patents. Paratoluensulfonic acid, especially when gradually added in one single dose or in portions, has proved particularly effective and at relatively low temperatures (below 40° C).

Before using the resulting peroxide mixture, it must be carefully washed, for instance with an alkali metal solution. It is important that the synthesis reaction be performed under an inert atmosphere, for instance under nitrogen. The washed peroxidic mixture must be concentrated, thus removing low boiling compounds (essentially diisopropylbenzene and cumene), preferably by steam distillation. In some cases, it is possible to add a peroxide of formula (III) directly to the peroxide mixture obtained, following the above mentioned operations.

In order that the invention may be well understood, the following Examples are given by way of illustration only. In the examples all parts and percentages are by weight unless otherwise stated.

In the examples reference will be made to the accompanying drawing which is a graph showing an ODR curve for a polymer cross-linked with a composition of the invention.

EXAMPLE 1

Benzene was alkylated with two mols of propylene, in the presence of $AlCl_3$ and according to usual Friedel-Crafts techniques, to give a mixture of diisopropylbenzenes. This mixture was then oxidized, to give

an oxidized mixture (containing para-di-isopropylbenzene, meta-diisopropylbenzene, para-di-isopropylbenzene monohydroperoxide, para-di-isopropylbenzene dihydroperoxide, meta-diisopropylbenzene monohydroperoxide and meta-di-isopropylbenzene dihydroperoxide). The oxidized mixture was then reduced with sodium hydrosulfide (NaHS), according to usual techniques, so that the hydroperoxy groups (-O-O-H) were converted into alcoholic groups (hydroxy groups). The resulting reduced mixture was then distilled to give a rectified mixture containing 84.8% by weight of a mixture of isopropyl-cumyl monoalcohols (35% of para and 65% of meta) and 4.7% by weight of isopropyl-cumyl di-alcohols (35% para and 65% meta).

135 g of the rectified mixture were loaded into a glass round-bottomed flask provided with a stirrer, thermometer and vacuum tap. Then, 171.7 g of a cumene mixture containing 78.5% by weight of cumene hydroperoxide, 7% by weight of cumyl alcohol and 10% by weight of nonoxidized cumene were added to the flask so that there were present:

(a) 0.632 mol of mono-alcohol + 0.032 mols of di-alcohol;

(b) 0.886 mol of cumene hydroperoxide + 0.088 mol of cumyl alcohol.

Thereafter, 71 g of an aqueous solution containing 70% by weight of para-toluenesulfonic acid were added, gradually over 30 minutes, at 25-85 °C and under nitrogen and the mixture was allowed to react, at 25-28 °C, for 3.5 hrs, with stirring. After the end of the reaction, the acidic aqueous phase was separated in a separating funnel and 2 washings were carried out, using each time 200 $cm^3$ (at 60 °C) of an aqueous 10% NaOH solution, followed by another two washings, using each time (at 60 °C), 200 $cm^3$ of deionized water. After having removed the cumene, by steam distillation, and after having dehydrated under vacuum at 65 °C, the resulting peroxide mixture contained oxygen in an amount of 4.7% by weight and had a purity degree of 91% by weight (total peroxidic content), a density of 0.98 $g/cm^3$ and a viscosity (at 25 °C) of 126 mPa.s Half life values of the mixture (thermal decomposition) were 113 °C (after 10 hours) and 188 °C (after 1 minute). Analysis showed that the mixture contained:

| | |
|---|---|
| di (cumylperoxy)-diisopropylbenzene (meta/para) (PEROXIMON 169) | 4% |
| dicumyl-peroxide (DCP) | 13% |
| isopropylcumyl-cumyl peroxide (meta/para) | 75% |

The remaining 10% comprised hydrocarbons and/or unreacted alcohols. (Isopropylcumyl-cumylperoxide is known in the market as PEROXIMON 168).

EXAMPLE 1a

100 parts of an ethylene-propylene elastomeric copolymer (trade name DUTRAL COO 54) were mixed with 0.3 parts of sulfur, 5 parts of ZnO and 50 parts of carbon black. To the mix there were added 3.46 parts of the peroxide mixture of Example 1 and the resulting blend was homogenized in a calender. The product obtained from the calender had the following properties:

(a) data of the ODR curve at 170 °C (oscillation arc = 3 °; oscillation frequency = 100 cycles/minute; see fig. 1);

MH = 82.6 inch pounds (95.2 cm.kg)
$ts_{10}$ = 102 seconds
$t_{90}$ = 456 seconds.

An ODR curve (Oscillating Disc Rheometer) has a course of the type shown in the drawing and is plotted by the aid of a rotating disc rheometer, according to ASTM-D-2084-71T.

On the absissa there are given times and on the ordinate the twisting torque (inch pounds, measured by means of a dynamometer) opposed by the polymer to the rotation of the disc; in the present case the highest cross-link density is revealed by the highest value of the torque (MH=82.6 inch pounds; 95.1 cm.kg) which does not vary further with time. The expressions $t_{90}$ and $ts_{10}$ respectively represent the time necessary to reach a twisting moment equal to 90% of the highest twisting moment and the time necessary to reach a level of 10 inch pounds above the lowest point of the ODR curve. As to other details reference is made to U.S. Patent 4,015,058

(b) "scorching" times at the Mooney viscosimeter (at 135 °C:

$ts_{10}$ = 738 seconds

$ts_{15}$ = 900 seconds

By "scorching" there is meant the untimely vulcanization which takes place (undesirably) during the extrusion of the blend, before its outlet from the die; this premature vulcanization often causing a shut down of operations.

By scorching time $ts_{10}$ or $ts_{15}$ (at the Mooney viscosimeter) there is meant the time necessary to reach an increase above of the lowest value of the viscosity equal to 10 or 15 Mooney units respectively. The viscosity is determined by means of a cutting disc Mooney viscosimeter (see ASTM D 1646-81).

EXAMPLE 2

18.8 g of the rectified alcohol of Example 1 were loaded into a glass round-bottom flask, provided with a stirrer, together with 74 g of a mixture containing 85% of cumyl alcohol and 10% of cumene (obtained by reducing 78% commercial cumene hydroperoxide); thereafter 36 g of dialcohol of diisopropylbenzene (solid; 65% meta + 35% para) and finally, 205 g of 78.5% by weight of commercial cumene, hydroperoxide, containing 7% by weight of cumyl alcohol and 10% by weight of cumene, were added. In total there were present:

0.09 mol of m/p-diisopropylbenzene monoalcohol;
0.19 mol of the m/p-diisopropylbenzene dialcohol;
0.568 mol of cumyl alcohol;
0.233 mol of cumene;
1.059 mol of cumene hydroperoxide.

Thereafter 71 g of a 70% aqueous solution of para-toluensulfonic acid were added over 30 minutes; then the mixture was reacted for 3 hours, the temperature being maintained between 25°C and 28°C. By further working as in Example 1, 210 g of a liquid product were obtained, having the following composition:

- alpha, alpha'-bis(cumylperoxy) diisopropylbenzene (PEROXIMON 169) =33%
- dicumylperoxide (DCP) =57%
- isopropylcumyl-cumylperoxide (PEROXIMON 168) = 10.3%

This product had an active $O_2$ content of 6.1%; a freezing point below -5°C and a viscosity at 20°C of 50 mPas. Half life values of the mixture (thermal decomposition) were 116°C (at 10 hours) and 179°C (at 1 minute).

EXAMPLE 2a

100 parts of a polyethylene blend, suitable for cable insulation, manufactured by B.P. Chemicals Co. under the trade name "HF NM 4993" were mixed with 2.5 parts of the peroxide mixture of Example 2 and the resulting mixture was homogenized in a calender. The product from the calender was moulded by compression and tested on the ODR rheometer at 180°C (oscillation arc = 5°) to give the following results:

MH = 78 inch pounds (89.9 cm. kg);
$t_{s2}$ = 66 seconds; $t_{90}$ = 288 seconds.

As described in Example 1, $t_{s2}$ indicates the time necessary to reach a level of 2 inch pounds (2.3 cm.kg) above the lowest point of the ODR curve.

EXAMPLE 3 (Comparative Example)

250 g of a mixture containing 57% of cumene hydroperoxide, 30% of cumyl alcohol and 10% of cumene, obtained by the partial reduction of commercial cumene hydroperoxide (78.5%) were loaded into a round-bottom flask provided with a stirrer. Then 37 g of a meta/para mixture (65% meta and 35% para) of diisopropylbenzene dialcohol were introduced. In total 0.94 mol of cumene hydroperoxide, 0.55 mol of cumyl alcohol and 0.19 mol of meta/para diisopropylbenzene di-alcohol were present. Then, 71 g of a 70% aqueous solution of para-toluensulfonic acid were introduced over 30 minutes with stirring, while keeping the reaction temperature between 25 and 30°C; the mixture was then allowed to react at this temperature for 3 hours. By working up as in Examples 1 and 2, 190 g of a product (liquid at room temperature), containing 60% of dicumyl peroxide and 35% of di(cumylperoxy)diisopropylbenzene were obtained. 21 g of tert.butylperoxide were added to the mixture to give a product containing 32% of di(cumylperoxy)-diisopropylbenzene (PEROXIMON 169), 54% by weight of dicumylperoxide (DCP) and 10% of tert.butyl-cumylperoxide. This liquid composition had the following characteristics:

- freezing point: lower than -5°C;

- viscosity at 20°C: 45 mPas;
- active oxygen content: 6.2%.

Half life times were 117°C (at 10 hours) and 181°C (at 1 minute). (Tert.-butyl-cumylperoxide is commercially known as "PEROXIMON 166").

EXAMPLE 3a (Comparative Example)

100 Parts of the polyethylene blend used in Example 2a were mixed with 2.5 parts of the peroxide mixture of Example 3 and the resulting blend was homogenized in a calender. By working as in Example 2a the following results were obtained:

MH = 85 inch-pounds (87.9 km.kg)

$t_{s2}$ = 54 seconds; $t_{90}$ = 300 seconds.

As described in U.S. patent 4,015,058, the quality of a peroxidic composition can be easily evaluated by calculating an efficiency factor (E) according to the equation:

$$E = (MH.t_{s2}) : (t_{90}-t_{s2})$$

The following table gives the efficiency factors obtained in the Examples:

| Ex | COMPOSITION OF THE PEROXIDIC MIXTURE | | | | E = $\frac{MH.\ t_{s2}}{t_{90}-t_{s2}}$ |
|---|---|---|---|---|---|
| | PEROXIMON 169 | DCP | PEROXIMON 168 | PEROXIMON 166 | |
| 2/a | 33% | 57% | 10% | - | 23,19 |
| 3/a | 33% | 57% | - | 10% | 18,65 |
| 4 | 2% | 57% | 41% | - | 13,25 |

In Examples 2a and 3a the presence of PEROXIMON 169, on which the invention is based, gives an efficiency much higher in comparison with Example 4, where the diperoxide is almost completely replaced by a monoperoxide (PEROXIMON 168).

EXAMPLE 4

Example 3a was repeated by replacing almost all the diperoxide of formula (III), by a comparable amount of PEROXIMON 168, thus obtaining the following results:

MH = 50 inch pounds (57.6 cm.kg)

$t_{s2}$ = 66 seconds; $t_{90}$ = 315 seconds.

## Claims

**1.** A liquid organic peroxide composition characterized in that it contains

(a) from 1 to 50 parts by weight of a diperoxide of the formula:

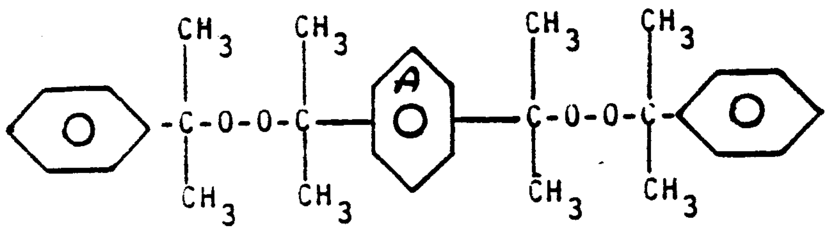 (II)

in which the two substituent groups on the central aromatic ring A are in the meta and/or para positions;
(b) from 5 to 75 parts by weight of dicumyl-peroxide; and
(c) from 1 to 85 parts by weight of a peroxide of the formula:

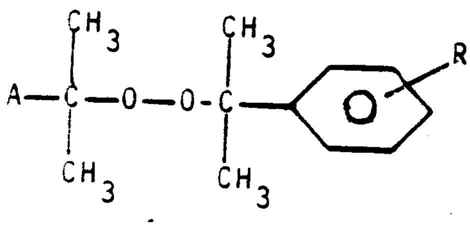 (III)

wherein R is an isopropyl group, and A is a methyl or phenyl group.

**2.** A composition as claimed in claim 1 characterized in that the meta : para isomer ratio of the diperoxide of formula (II) is from 1.2 : 1 to 2.5 : 1, preferably from 1.5 : 1 to 2.1 : 1.

**3.** A composition as claimed in claim 1 or claim 2 characterized in that it contains from 50 to 70 parts by weight of dicumyl-peroxide.

**4.** A composition as claimed in any one of the preceding claims characterized in that the meta/para isomer ratio for the peroxide of formula (III) is from 1.2 : 1 to 2.5:1, preferably from 1.5:1 to 2.1:1.

**5.** A composition as claimed in any one of the preceding claims characterized in that it contains:-

(a) from 20 to 45 parts by weight of the diperoxide of formula (II), wherein the meta/para isomeric ratio is from 1.2 to 2.5;
(b) from 50 to 70 parts by weight of dicumyl-peroxide; and
(c) from 1 to 25 parts by weight of a peroxide of the formula:

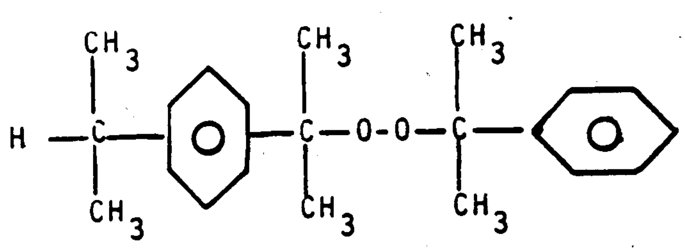

**6.** The use of the compositions as claimed in any one of the preceding claims for the cross-linking of thermoplastic and/or elastomeric polymers and copolymers, especially polyethylene polymers and

copolymers, ethylene-propylene copolymers and ethylene/vinylacetate copolymers.

**7.** A process for the manufacture of a composition according to claim 1, characterized in that it comprises the steps of:

(a) oxidising meta- and/or para-di-isopropyl-benzene to give an oxidized mixture containing isopropylcumyl mono-hydroperoxide and up to 12% by weight (based on the mono-derivative) of the corresponding di-hydroperoxide;
(b) reducing the oxidized mixture, to give a reduced product wherein hydroxyl groups (alcoholic groups) replace the hydroperoxidic groups in the oxidized mixture;
(c) optionally distilling the reduced mixture to give a rectified mixture, containing higher ratios of isopropylcumyl mono-alcohol and up to 30% by weight of the corresponding di-alcohol;
(d) optional admixture with additional amounts of cumyl alcohol and/or m/p-diisopropylbenzene dialcohol, so that the amounts of dicumyl-peroxide and/or of the diperoxide of formula (II) in the final liquid peroxidic composition are increased; and
(e) reaction with a cumenic mixture, containing a higher amount of cumene hydroperoxide and up to 10% by weight (based on the hydroperoxide) of cumyl alcohol.

**8.** A process as claimed in claim 7, characterised in that reaction (e) is performed in the presence of para-toluen-sulfonic acid, at a temperature of 40° C or below.

**Revendications**

**1.** Une composition organique liquide à base de peroxyde caractérisée en ce qu'elle contient:
(a) de 1 à 50 parties en poids d'un diperoxyde de formule:

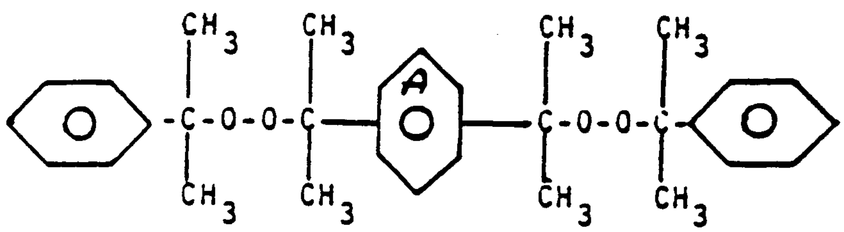

(II)

dans laquelle les deux substituants sur le cycle aromatique central A sont en position méta et/ou para;
(b) de 5 à 75 parties en poids de peroxyde de dicumyle; et
(c) de 1 à 85 parties en poids d'un peroxyde de formule:

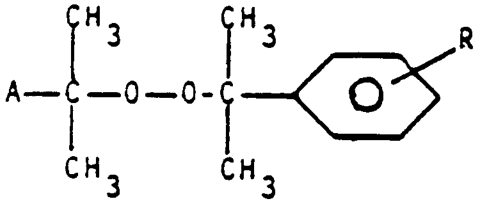

(III)

dans laquelle:
R est un radical isopropyle; et
A est un radical méthyle ou phényle.

**2.** Une composition selon la revendication 1, caractérisée en ce que le rapport isomérique méta/para du diperoxyde de formule (II) est compris entre 1,2/1 et 2,5/1, et de préférence entre 1,5/1 et 2,1/1.

**3.** Une composition selon les revendication 1 ou 2, caractérisée en ce qu'elle contient entre 50 et 70 parties en poids de peroxyde de dicumyle.

**4.** Une composition selon l'une quelconques des revendications précédentes, caractérisée en ce que le rapport isomérique méta/para du diperoxyde de formule (III) est compris entre 1,2/1 et 2,5/1, et de préférence entre 1,5/1 et 2,1/1.

**5.** Une composition selon l'une quelconques des revendications précédentes, caractérisée en ce qu'elle contient:
(a) de 20 à 45 parties en poids du diperoxyde de formule (II), dans lequel le rapport isomérique méta/para est compris entre 1,2 et 2,5;
(b) de 50 à 70 parties en poids de peroxyde de dicumyle; et
(c) de 1 à 25 parties en poids d'un peroxyde de formule:

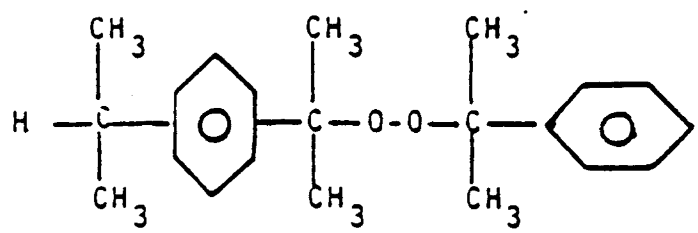

**6.** L'utilisation des compositions selon l'une quelconque des revendications précédentes pour la réticulation de polymères et de copolymères thermoplastiques et/ou élastomères, et en particulier de polymères et de copolymères de polyéthylène, de copolymères éthylène/propylène et de copolymères éthylène/acétate de vinyle.

**7.** Un procédé de fabrication d'une composition selon la revendication 1, caractérisé en ce qu'il comprend les étapes de:
(a) oxydation du méta- et/ou para-diisopropylbenzène pour obtenir un mélange oxydé contenant du monohydroperoxyde d'isopropylcumyle et jusqu'à 12% en poids (par rapport au monohydroperoxyde) du dihydroperoxyde correspondant;
(b) réduction du mélange oxydé pour obtenir un produit réduit dans lequel des groupements hydroxyle (groupements alcool) remplacent les groupements hydroperoxydes du mélange oxydé;
(c) distillation éventuelle du mélange réduit pour obtenir un mélange rectifié, contenant une proportion plus importante de monoalcool isopropylcumylique et jusqu'à 30% en poids du dialcool correspondant;
(d) addition éventuelle de quantités supplémentaires d'alcool cumylique et/ou de dialcool m/p-diisopropylbenzénique, de façon à augmenter la quantité de peroxyde de dicumyle et/ou du diperoxyde de formule (II) dans la composition liquide finale à base de peroxyde; et
(e) réaction avec un mélange cuménique, contenant une proportion importante d'hydroperoxyde de cumène et jusqu'à 10% en poids (par rapport à l'hydroperoxyde) d'alcool cumylique.

**8.** Un procédé selon la revendication 7, caractérisé en ce que la réaction (e) s'effectue en présence d'acide paratoluènesulfonique, à une température inférieure ou égale à 40 °C.

## Patentansprüche

**1.** Flüssige organische Peroxidmischung, dadurch gekennzeichnet, daß sie
(a) 1 bis 50 Gew.-% eines Diperoxids der Formel

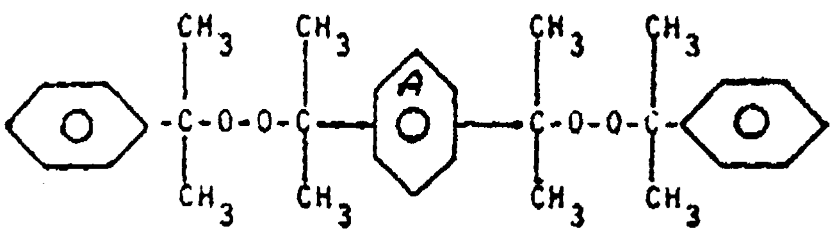 (II),

in welcher sich die beiden Substituenten am mittleren aromatischen Ring A in meta und/oder para-Stellung befinden,

(b) 5 bis 75 Gew.-% Dicumylperoxid und

(c) 1 bis 85 Gew.-% eines Peroxids der Formel

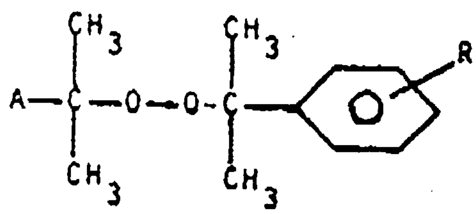 (III),

in welcher R eine Isopropylgruppe bedeutet und A eine Methyl- oder Phenylgruppe ist,

enthält.

**2.** Zusammensetzung nach Anspruch 1, dadurch gekennzeichnet, daß das Verhältnis von meta- zu para-Isomer des Diperoxids nach Formel II von 1,2 : 1 bis 2,5 : 1, vorzugsweise von 1,5 : 1 bis 2,1 : 1, beträgt.

**3.** Zusammensetzung nach Anspruch 1 oder 2, dadurch gekennzeichnet, daß sie 50 bis 70 Gew.-% Dicumylperoxid enthält.

**4.** Zusammensetzung nach irgendeinem der Vorhergehenden Ansprüche, dadurch gekennzeichnet, daß das Verhältnis von meta- zu para-Isomer für das Peroxid gemäß Formel III von 1,2 : 1 bis 2,5 : 1, vorzugsweise von 1.5 : 1 bis 2,1 : 1, beträgt.

**5.** Zusammensetzung nach irgendeinem der vorhergehenden Ansprüche, dadurch gekennzeichnet, daß sie

(a) 20 bis 45 Gew.-% des Diperoxids nach Formel II mit einem meta/para-Isomerenverhältnis von 1,2 bis 2,5,

(b) 50 bis 70 Gew.-% Dicumylperoxid und

(c) 1 bis 25 Gew.-% eines Peroxids der Formel

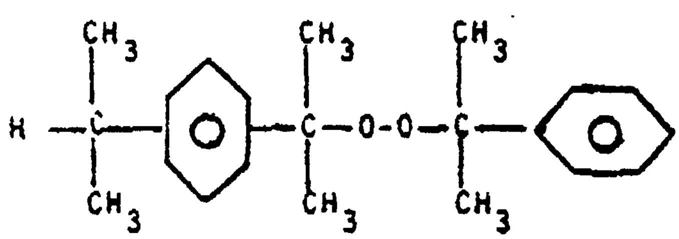

enthält.

**6.** Verwendung der Zusammensetzungen nach irgendeinem der vorhergehenden Ansprüche zur Quervernetzung von thermoplastischen und/oder elastomeren Polymeren und Copolymeren, insbesondere Polyäthylen-Polymeren und -Copolymeren, Äthylen-Propylen-Copolymeren und Äthylen/Vinylacetat-Copolymeren.

**7.** Verfahren zur Herstellung einer Zusammensetzung nach Anspruch 1, dadurch gekennzeichnet, daß es die folgenden Schritte umfaßt:

(a) Oxidieren von meta- und/oder para-Diisopropylbenzol zu einer oxidierten Mischung, die Isopropylcumylmonohydroperoxid und bis zu 12 Gew.-% (bezogen auf das Mono-Derivat) des entsprechenden Dihydroperoxids enthält,

(b) Reduzieren der oxidierten Mischung zu einem reduzierten Produkt, in dem die Hydroxylgruppen (alkoholische Gruppen) die Hydroperoxidgruppen in der oxidierten Mischung ersetzen,

(c) gegebenenfalls Destillieren der reduzierten Mischung zu einer rektifizierten Mischung, die höhere Anteile an Isopropylcumyl-mono-alkohol und bis zu 30 Gew.-% des entpsrechenden Dialkohols enthält,

(d) gegebenenfalls Zusammenmischen mit zusätzlichen Mengen Cumylalkohol und/oder m/p-Diisopropylbenzoldialkohol, so daß die Mengen von Dicumylperoxid und/oder des Diperoxids von Formel II in der endgültigen flüssigen Peroxidmischung erhöht sind und

(e) Umsetzen mit einer Cumolmischung, die einen höheren Anteil an Cumolhydroperoxid und bis zu 10 Gew.-% (bezogen auf das Hydroperoxid) Cumylalkohol enthält.

**8.** Verfahren nach Anspruch 7, dadurch gekennzeichnet, daß die Umsetzung nach (e) in Gegenwart von para-Toluolsulfonsäure bei einer Temperatur von 40 °C oder darunter durchgeführt wird.

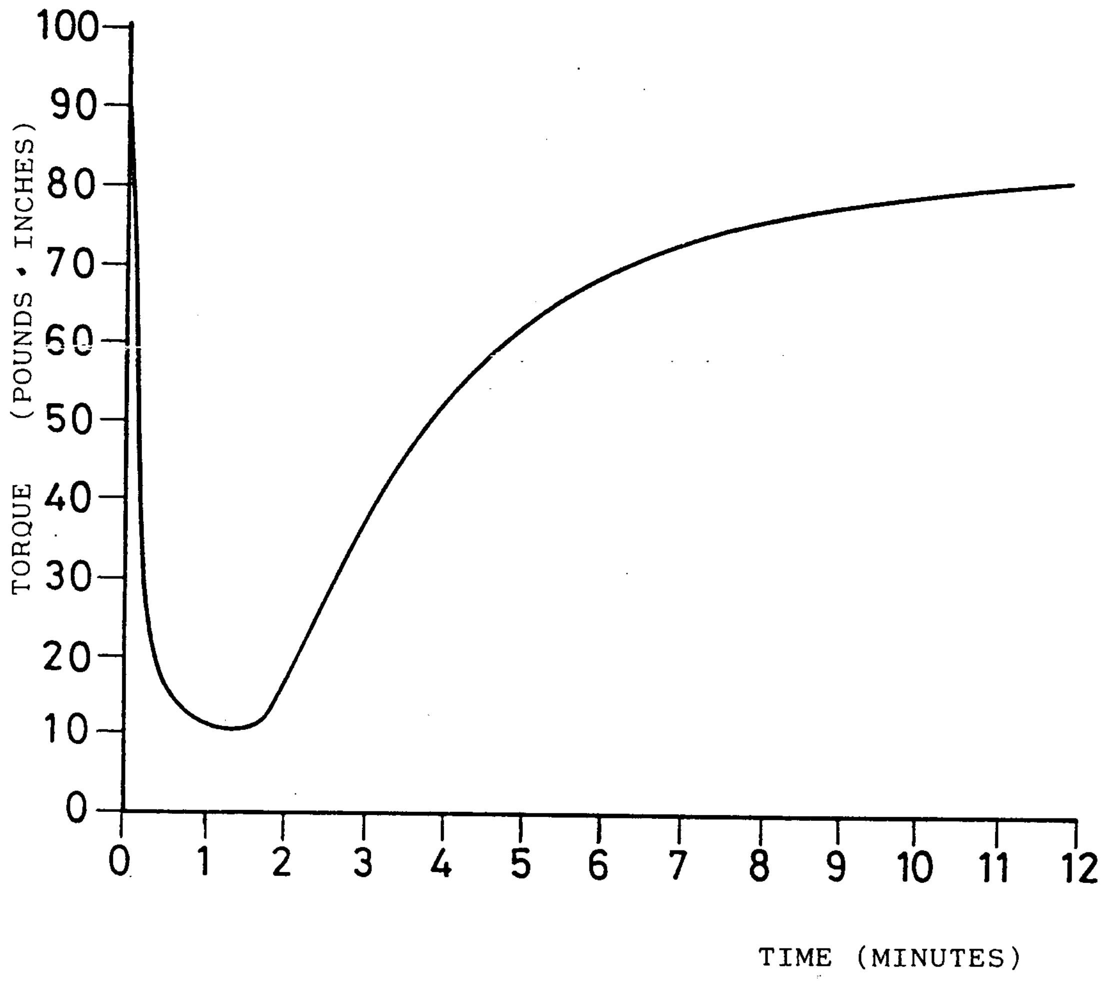
TORQUE (POUNDS • INCHES)
100
90
80
70
60
50
40
30
20
10
0
0
1
2
3
4
5
6
7
8
9
10
11
12
TIME (MINUTES)